# EUROPEAN PATENT APPLICATION

(11) **EP 2 371 358 A1**
(43) Date of publication of application: **05.10.2011**
(21) Application number: 11002556.6
(22) Date of filing: 28.03.2011
(51) Int. Cl.: A61K 9/70, A61K 31/192, A61K 31/4045, A61K 31/405

(54) **Film-form preparation and method for producing the same**

(30) Priority: 30.03.2010 JP 2010079429
(71) Applicant: Nitto Denko Corporation, Ibaraki-shi Osaka 567-8680 (JP)
(72) Inventor: Asari, Daisuke, Ibaraki-shi Osaka 567-8680 (JP); Hori, Mitsuhiko, Ibaraki-shi Osaka 567-8680 (JP); Shishido, Takuya, Ibaraki-shi Osaka 567-8680 (JP)
(74) Representative: Müller-Boré & Partner Patentanwälte

(57) **Abstract**

The present invention provides a film-form preparation having a rapid dissolution profile in the mouth and sufficient film strength, and also having excellent appearance and feel. More specifically, the present invention provides a film-form preparation including: a water-soluble edible polymer; and water-insoluble drug particles, wherein an average particle size of the drug particles is 0.1 to 60 µm.

## Description

### TECHNICAL FIELD

The present invention relates to a film-form preparation (film-form medication) that readily dissolves in the mouth. More specifically, the present invention relates to a film-form preparation wherein drug particles for oral administration are dispersed such that the drug will dissolve rapidly in the mouth and be absorbed via the gastrointestinal tract or oral mucosa, and a method for producing the same.

### BACKGROUND ART

At present orally administered drugs are marketed as uncoated tablets, coated tablets, capsules, powders, granules, liquids, etc.
Orally disintegrating tablets and rapidly dissolving oral films are already on the market as medications that disintegrate in the mouth and are absorbed by the gastrointestinal tract. Among these a film-form preparation is useful from the standpoint of rapid dissolution.

A considerable amount of research has been conducted on such film-form preparations. For example, Patent Document 1 discloses a film-form preparation comprising hydroxypropyl cellulose or a mixture of hydroxypropyl cellulose and polyvinyl pyrrolidone, a tannin, and a drug.
Patent Document 2 discloses a film-form preparation comprising a drug and low-substituted hydroxypropyl cellulose.
Patent Document 3 discloses a film-form preparation comprising a drug and hydroxypropyl cellulose.
Patent Document 4 discloses a film-form preparation comprising a drug and hydroxypropyl cellulose.
Patent Document 5 discloses a tablet obtained by drying a liquid suspension wherein a drug and polyvinyl pyrrolidone are dissolved or dispersed in an organic solvent.
Patent Document 6 discloses that a film-form preparation containing a drug can also contain polymers that are water-soluble, water-swellable, water-insoluble, or a combination thereof.
Patent Document 7 discloses a film-form preparation containing drug particles.

However, the appearance and physical properties such as feel, etc., of film-form preparations are still unsatisfactory because the drugs contained in prior art film-form preparations exist almost entirely in a dissolved state or, even if they are in a solid state, they have been dissolved and recrystallized in the preparation to obtain the solid state. Even in film-form preparations wherein the drug is present in a solid state, it has been almost impossible to contain the drug therein in a particulate state, let alone control the particle size, because the drug is dissolved at least once during the manufacturing process.

### PRIOR ART DOCUMENT

### PATENT DOCUMENT

Patent Document 1: JP-B 3496727
Patent Document 2: JP-A 2008-169138
Patent Document 3: JP-A 2004-43450
Patent Document 4: JP-T 2007-528876
Patent Document 5: JP-A H11-116465
Patent Document 6: WO 2004/066986
Patent Document 7: JP-T 2002-523359

### SUMMARY OF THE INVENTION

### PROBLEMS TO BE SOLVED BY THE INVENTION

In light of the above, an object of the present invention is to provide a film-form preparation with a rapid dissolution profile in the mouth and sufficient film strength, and with excellent appearance and feel, and a method for producing the same.

### MEANS FOR SOLVING THE PROBLEMS

After thorough and incisive investigation of the above problem, the inventors discovered that a film-form preparation with a rapid dissolution profile in the mouth and sufficient film strength, and with excellent appearance and feel can be obtained using water-insoluble drug particles as the drug particles and a water-soluble edible polymer into the film-form preparation, thus completing the present invention.

More specifically, the present invention is a film-form preparation including: a water-soluble edible polymer; and water-insoluble drug particles, wherein an average particle size of the drug particles is 0.1 to 60 µm.
In the film-form preparation of the present invention, a particle size of the drug particles is preferably 0.1 to 30 µm_{.}
In addition, the edible polymer is preferably a solid at normal temperatures.
In addition, the edible polymer is preferably at least one type selected from the group consisting of polyvinyl pyrrolidone, hydroxypropyl cellulose, hydroxypropyl methylcellulose, and pullulan.
In addition, a weight-average molecular weight of the polyvinyl pyrrolidone preferably ranges from 2,500 to 3,000,000.
In addition, a weight-average molecular weight of the hydroxypropyl cellulose preferably ranges from 10,000 to 1,200,000.
In addition, the hydroxypropyl cellulose preferably has a hydroxypropoxy group-substitution degree of 50 to 100%.
In addition, a weight-average molecular weight of the hydroxypropyl methylcellulose preferably ranges from 10,000 to 1,500,000.
In addition, the hydroxypropyl methylcellulose preferably has a hydroxypropoxy group-substitution degree of 4 to 32%, and a methoxy group-substitution degree of 16 to 30%.
In addition, a molecular weight of the pullulan preferably ranges from 10,000 to 2,000,000.
The present invention is also a method for producing a film-form preparation including a water-soluble edible polymer and water-insoluble drug particles, the drug particles having an average particle size of 0.1 to 60 µm, the method including: preparing a liquid dispersion of a drug containing the edible polymer, the drug particles, and water; forming the liquid dispersion of the drug into a thin layer; and drying the thin layer.
The present invention is described in greater detail below.

The film-form preparation of the present invention contains an edible polymer that is soluble in water and drug particles that are insoluble in water.
With regard to solubility in water, when 100 mL of water or more is required to dissolve 1 g of solute at 20°C, then the solute "does not dissolve in water," and the expression "insoluble in water (or water-insoluble)" is used in this description, and when less than 5 mL of water is sufficient to dissolve 1 g of solute at 20°C, then the solute "dissolves in water," and the expression "soluble in water (or water-soluble)" is used in this description. When the amount of water sufficient to dissolve 1 g of solute is less than 3 mL, then the expression "very soluble" is used in this description.

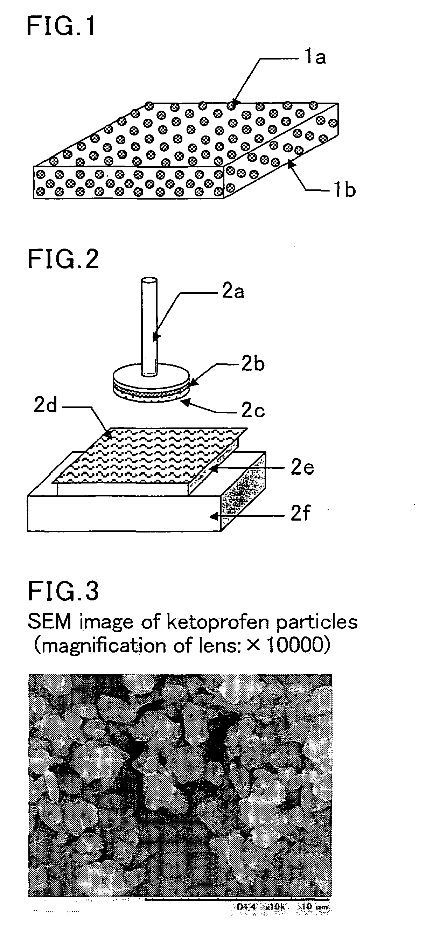

Figure 1 is a schematic drawing showing one example of an embodiment of the film-form preparation of the present invention, and as shown in Figure 1, in the film-form preparation of the present invention, the water-insoluble drug particles 1a are dispersed in a base material 1b comprising the water-soluble edible polymer.

The drug particles can be localized to the surface of the base material or to a specific place therein, but preferably they are dispersed uniformly throughout the base material. Dispersing the drug particles uniformly in the base material enables rapid release of the drug in the mouth and improves the physical properties.of the film-form preparation. Therefore, in the film-form preparation of the present invention the desired drug release properties for the drug particles can be attained without the need to coat the drug particles (e.g., encapsulate the drug particles in microcapsules).

The thickness of the film-form preparation of the present invention is not particularly limited herein, but a range of 30 to 300 µm, for example, is preferred. If the thickness is less than 30 µm, problems can arise from the standpoint of film strength of the film-form preparation and product handling properties; if the thickness exceeds 300 µm, the film-form preparation will require more time to dissolve in the mouth, and may not dissolve easily.
The planar shape of the film-form preparation of the present invention is not particularly limited herein, and it can be made into a desired shape such as a rectangle, square, circle, etc.

The drug particles used in the present invention have the solubility property of being insoluble in water.
In the film-form preparation of the present invention, drug particles having the above solubility property are used as the drug particles, whereas a water-soluble polymer described below is used as the edible polymer wherein the drug particles are dispersed. Using a combination of drug particles and edible polymer with such solubility properties and using water as the liquid medium during manufacture can facilitate containing the drug particles in a particulate state within the film-form preparation of the present invention and controlling the particle size thereof. By following the criteria below, for example, a person skilled in the art can easily and clearly discern the difference between drug particles in a recrystallized state and drug particles in a particulate state in a film-form preparation.
In other words, drug particles contained in a particulate state in a film-form preparation have irregular and nonuniform shapes and sizes, and are sometimes referred to as amorphous because they are organized spontaneously within the film-form preparation. Conversely, drug particles contained in a recrystallized state in a film-form preparation have an artificially manipulated shape and size because the manufacturer controls the particle size during manufacture.

Preferably, the drug particles are a solid at normal temperatures. When they are a solid at normal temperatures, the drug particles in the film-form preparation of the present invention can easily form a particulate state. The term "solid at normal temperatures" means a lack of liquidity at 20°C.

The average particle size of the drug particles in the film-form preparation of the present invention is 0.1 to 60 µm. When the average particle size is less than 0.1 µm, individual drug particles may agglomerate and the flexibility of the film-form preparation can become nonuniform in places. When the average particle size exceeds 60 µm, the flexibility can also become nonuniform in places if the particles are contained in a film-form preparation of practical thickness.
Preferably, the average particle size of the drug particles is 0.1 to 30 µm. Having the average particle size in this range enables the preparation of a film-form preparation with uniform strength and flexibility at a practical thickness.
Here the term average particle size refers to the average particle size of the equivalent circular diameters of 50% of the particles by volume. The term equivalent circular diameter refers to the equivalent circular diameter of the projected area. More specifically it is the diameter of a circle with an area equal to that of the projection of the particle at plane, and is also referred to as the Heywood diameter.
If the average particle size of the drug particles lies outside the above range, sized particles falling within the above range can be used. Adjustment of the average particle size can be performed by pulverization, dry pulverization, granulation using wet granulation, etc., classification using a sieve, mechanical classifier, etc.

Preferably, the drug particles are prepared by granulating from the standpoint of physical properties and appearance of both the drug particles and the film-form preparation. Publicly known means, for example spray drying, jet milling, etc., can be noted as the technical means for granulating.
In addition, from the standpoint of physical properties and appearance of the film-form preparation, the drug particles do not need to be microencapsulated. Drug particles that are not microencapsulated are preferable from the standpoint of rapid dissolution.

In the present invention, the term drug particle refers to a mass of solid drug.
Such drug particles are not particularly limited in the present invention provided they have the above solubility properties and can be administered orally. Concrete examples of such drugs include sedatives, expectorants, laxatives, anticancer drugs, antidiabetic drugs, anti-Parkinson's drugs, antidepressants, tranquilizers, anti-dementia drugs, antihypertensive drugs, anti-hyperlipidemia drugs, anti-migraine drugs, therapeutic agents for osteoporosis, therapeutic agents for hypotension, antitussive drugs, therapeutic agents for digestive ulcers, therapeutic agents for frequent urination and voiding disorders, therapeutic agents for urinary incontinence, anti-ulcer drugs, allergy drugs, 5-HT3 receptor antagonists (antiemetics), and the like.

Even more specifically, ketoprofen particles, melatonin particles, indomethacin particles and the like can be noted as examples of the above drug particles.

Drug particles that do not taste bitter are preferred, but drug particles that do taste bitter can also be suitably used by performing a bitterness masking technique, for example, microencapsulation, or by adding a bitterness blocking agent, sweetener, flavoring, or fragrance.

The content of the drug particles will differ depending on the properties, etc., thereof, but preferably the content will be 0.1 to 80 wt% of the total content of solids contained in the film-form preparation of the present invention. When the content is less than 0.1 wt%, a rapid dissolution profile in the mouth and sufficient film strength may not be obtained. Moreover, a clear improvement over a film prepared by dissolving the drug may not be seen with regard to the gummy sensation in the mouth and sticky sensation when touched by the fingers resulting from the water-soluble polymer. However, this does not present a problem from a practical standpoint. On the other hand, when the content of drug particles exceeds 80 wt%, problems may occur in the shape retention properties, etc., of the product unless the particle size of the drug particles is made very small. A more preferred upper limit is 60 wt%. By making the content 60 wt% or less, the above advantageous effect of the present invention can be more properly obtained.

The edible polymer is a component constituting the base material of the film-form preparation of the present invention, and it is a polymer capable of being formed into a film.
The edible polymer is not particularly limited herein provided it is soluble in water and is edible, but preferably it is a solid at normal temperatures.
Preferably, such an edible polymer has a molecular weight of 2,000 to 4,000,000. When the molecular weight is less than 2,000, the film-forming properties will be poor, and retaining the shape of the film-form preparation may be difficult. On the other hand, when the molecular weight exceeds 4,000,000, the solubility of the film-form preparation will become poor, and this may become a problem from a practical standpoint. A more preferred molecular weight is a range of 2,500 to 3,000,000.

More specifically, the edible polymer is preferably at least one type selected from the group consisting of polyvinyl pyrrolidone (hereinafter, PVP), hydroxypropyl cellulose (hereinafter, HPC), hydroxypropyl methylcellulose (hereinafter, HPMC), and pullulan.
These edible polymers are preferred because they exhibit sufficient solubility in water, and when used in a film-form preparation, dissolve rapidly in the mouth.
Among the above edible polymers, HPC, HPMC, and pullulan are more preferred because these edible polymers have less hygroscopicity with regard to relative humidity than PVP, and are considered preferable from a practical standpoint.

Preferably, the molecular weight of the above PVP is 2,500 to 3,000,000. When the molecular weight is less than 2,500, there is concern that formability, stability, and hygroscopicity will be adversely affected; conversely, when the molecular weight exceeds 3,000,000, there is concern that solubility will become poor. A more preferred molecular weight is 2,500 to 1,200,000, and 2,500 to 1,000,000 is even more preferred.
In this description, the term the molecular weight refers to weight-average molecular weight, and is obtained by gel permeation chromatography analysis.

Preferably, the molecular weight of the above HPC is 10,000 to 1,200,000. When the molecular weight is less than 10,000, there is concern that formability, hygroscopicity, and stability will be adversely affected, and when the molecular weight exceeds 1,200,000, there is concern that solubility will become poor. A more preferred molecular weight for the HPC is 10,000 to 370,000.

Preferably, the hydroxypropoxy group-substitution degree in the above HPC is 50 to 100%. When it is less than 50%, there is concern that the solubility thereof in water may become poor.
The method for measuring the hydroxypropoxy group-substitution degree follows the quantitative method described in the section entitled "Hydroxypropyl cellulose" in the Official Monographs of the Fifteenth Edition of the Japanese Pharmacopoeia. Preferably, the hydroxypropoxy group-substitution degree in the above HPC is at least 53.4%.

Preferably, the molecular weight of the above HPMC is 10,000 to 1,500,000. When the molecular weight is less than 10,000, there is concern that formability, stability, and hygroscopicity will be adversely affected, and conversely, when the molecular weight exceeds 1,500,000, there is concern that solubility will become poor. A more preferred molecular weight for the HPMC is 10,000 to 100,000.

Preferably, the hydroxypropoxy group-substitution degree in the above HPMC is 4 to 32%, and the methoxy group-substitution degree is 16 to 30%. If the hydroxypropoxy group and methoxy group degrees of substitution fall outside these ranges, there is concern that solubility in water will become poor.
More preferably, the hydroxypropoxy group-substitution degree in the above HPMC is 4 to 12%. A more preferred lower limit for the methoxy group-substitution degree in the above HPMC is 19%, and a more preferred upper limit is 30%.
The method for measuring the hydroxypropoxy group and methoxy group degrees of substitution follow the quantitative method described in the section entitled "Hypromellose" in the Official Monographs of the Fifteenth Edition of the Japanese Pharmacopoeia

Preferably, the molecular weight of the pullulan is 10,000 to 2,000,000. When the molecular weight is less than 10,000, there is concern that formability, hygroscopicity, and stability will be adversely affected, and when the molecular weight exceeds 2,000,000, there is concern that solubility will become poor. A more preferred molecular weight for the pullulan is 10,000 to 370,000.

The film-form preparation of the present invention can use a suitable amount of edible polymer other than the edible polymers described above in combination therewith.
Examples of the other edible polymer include synthetic polymers such as polyvinyl alcohol, carboxyvinyl polymer, hydroxypropyl methylcellulose, hydroxyethyl cellulose, methylcellulose, ethylcellulose, low-substituted hydroxypropyl cellulose, crystalline cellulose, carboxymethylcellulose sodium, carboxymethylcellulose calcium, carboxymethylcellulose, and carboxymethyl starch sodium; and polymers obtained from natural substances such as sodium alginate, dextran, casein, pectin, guar gum, xanthan gum, tragacanth gum, acacia gum, gum arabic, and starch.

The content of the edible polymer in the film-form preparation of the present invention is preferably 10 to 95 wt% in relation to the total weight of solids contained therein. When the content of edible polymer is less than 10 wt%, the content of the aforementioned drug particles in the film-form preparation of the present invention will become too great, and unless the particle size of the drug particles is made very small, there will be a problem with shape retention properties, etc., of the product. On the other hand, when the content exceeds 95 wt%, a rapid dissolution profile in the mouth and sufficient film strength may not be obtained. A more preferred content is 20 to 80 wt%.

In addition to the above materials, the film-form preparation of the present invention can also contain a suitable amount of fragrance, flavoring, sweetener, coloring, preservative, antioxidant, stabilizer, surfactant, plasticizer (polyethylene glycol (PEG), etc.) within a range that does not hinder the effect of the present invention.

The film-form preparation of the present invention can be manufactured, for example, by the following method: first the desired amounts of edible polymer and drug particles adjusted so that the average particle size is 0.1 to 60 µm by pulverization, granulation, a classifier, and the like, are prepared, and then water is added to prepare a liquid dispersion of the drug. Next, the film-form preparation of the present invention can be produced by spreading a suitable amount of the liquid dispersion of the drug on a release film to form a thin film thereon, and then drying the thin film. In addition, the dried thin film is cut to a desired size, and as needed, sealed and packaged to produce a product.
Such a method for producing the film-form preparation of the present invention also constitutes the present invention.
During the manufacturing process of the film-form preparation of the present invention, when preparing the liquid dispersion of the drug, if the drug particles are added after the full amount of edible polymer is dissolved in water, it may become difficult to disperse the drug particles sufficiently due to the viscosity of the polymer solution. As a result, in the method for producing the film-form preparation of the present invention it is preferable to first disperse the drug particles in water to prepare a liquid dispersion of the drug and then dissolve the edible polymer therein.

If bubbles form in the liquid during the preparation of the above liquid dispersion of the drug, it is preferable to let the dispersion stand overnight and perform degassing under vacuum. Furthermore, preferably water (purified water) is the only medium used in preparing the liquid dispersion of the drug, but very small amounts of ethanol, propanol, acetone, and the like, can also be added.

### EFFECTS OF THE INVENTION

The film-form preparation of the present invention can stably contain a sufficient quantity of drug expressing a rapid dissolution profile in the mouth because the drug particles are dispersed in a particulate state, and can have sufficient film strength, a satisfactory sensation when touched by the fingers, appearance, and the like.
Therefore, with the film-form preparation of the present invention it is possible for the drug particles to achieve the desired sustained-release capability of the drug without coating the drug particles, e.g., encapsulating them in microcapsules.
Furthermore, the production method for the film-form preparation of the present invention enables the drug particles to be dispersed and carried in a film-form preparation without the need to dissolve the drug in solution, and therefore a film-form preparation containing the drug in a particulate state can be produced efficiently, and the size and shape of the drug particles can be controlled thereby.

### BRIEF DESCRIPTION OF THE DRAWINGS

Figure 1 is a schematic drawing showing one example of an embodiment of the film-form preparation of the present invention;
Figure 2 is a schematic drawing showing the tack duration test;
Figure 3 is an SEM image of ketoprofen particles;
Figure 4 is an SEM image of melatonin particles A;
Figure 5 is an SEM image of melatonin particles B;
Figure 6 is an SEM image of melatonin particles C;
Figure 7 is an SEM image of indomethacin particles;
Figure 8 is a micrograph of the surface of the film-form preparation of Example 1;
Figure 9 is a micrograph of the surface of the film-form preparation of Example 2;
Figure 10 is a micrograph of the surface of the film-form preparation of Example 3;
Figure 11 is a micrograph of the surface of the film-form preparation of Example 4;
Figure 12 is a micrograph of the surface of the film-form preparation of Example 5;
Figure 13 is a micrograph of the surface of the film-form preparation of Example 6;
Figure 14 is a micrograph of the surface of the film-form preparation of Example 7;
Figure 15 is a micrograph of the surface of the film-form preparation of Example 8;
Figure 16 is a micrograph of the surface of the film-form preparation of Comparative Example 1;
Figure 17 is a micrograph of the surface of the film-form preparation of Comparative Example 2;
Figure 18 is a micrograph of the surface of the film-form preparation of Comparative Example 3;
Figure 19 is a micrograph of the surface of the film-form preparation of Comparative Example 4;
Figure 20 is a micrograph of the surface of the film-form preparation of Comparative Example 5; and
Figure 21 is a micrograph of the surface of the film-form preparation of Comparative Example 6;

### MODES FOR CARRYING OUT THE INVENTION

The present invention is described in detail through the following examples, but is by no means limited to those examples.

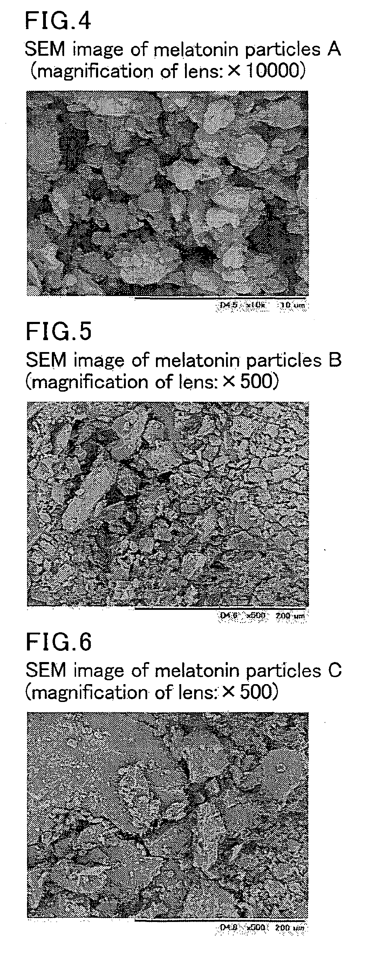

The various drug particles used in the Examples and Comparative Examples were obtained passing the powder through a 32 µm, 50 µm or 90 µm sieve after pulverization, or obtained fine particles by a jet mill (product of Hosokawa Micron Group, spiral jet mill model 50AS) or a spray dryer (product of Büchi Labortechnik AG, mini spray dryer model B-290) after pulverization. The particle sizes of these drug particles was measured by electron microscope (product of Hitachi High-Technologies Corp., model TM-1000) and the 50 vol% average particle size was calculated from the measurement results of 200 particles. This value was used as the particle size index of the particles.
Table 1 shows the 50 vol% average particle size and standard deviation of the drug particles. Images of these particles are shown in FIGS. 1 to 5.

**Table 1**

| Drug | 50 vol% average particle size [um] | Standard deviation [um] |
|---|---|---|
| Ketoprofen particles | 1.7 | 0.5 |
| Melatonin particles A | 2.1 | 0.6 |
| Melatonin particles B | 28.4 | 6.2 |
| Melatonin particles C | 180.5 | 69.4 |
| Indomethacin particles | 1.8 | 0.5 |

### (Example 1)

After 0.4 parts by weight of polyethylene glycol (PEG400) was added to 18.6 parts by weight of distilled water and stirred well, 6.7 parts by weight of HPC (product of Nippon Soda Co., Ltd., brand name: Nisso HPC SSL) with a molecular weight of approximately 30,000 and a hydroxypropoxy group-substitution degree of 53.4 to 77.5% was added, stirred, and dissolved using a rolling mixer. Then 3.0 parts by weight of previously sized ketoprofen particles were added and dispersed by sonication to prepare a liquid dispersion of the drug. After the liquid dispersion of the drug was adequately degassed, it was spread onto a polyester release film and dried to prepare a film with a thickness of approximately 70 µm. The resulting film was cut into 4 cm² rectangles to obtain the film-form preparation of Example 1.

### (Example 2)

The film-form preparation of Example 2 was obtained using the same procedure as in Example 1 except PVP (product of Wako Pure Chemical Industries Co., Ltd., reagent name: polyvinyl pyrrolidone K90) with a molecular weight of 1,050,000 to 1,200,000 was used in place of the HPC to make the composition shown in Table 2.

### (Example 3)

The film-form preparation of Example 3 was obtained using the same procedure as in Example 1 except HPMC (product of Shin-Etsu Chemical Co., Ltd., product name: TC-5E) with a molecular weight of 16,000, a hydroxypropoxy group-substitution degree of 7.0 to 12.0%, and a methoxy group-substitution degree of 28.0 to 30.0% was used in place of the HPC to make the composition shown in Table 2.

### (Example 4)

The film-form preparation of Example 4 was obtained using the same procedure as in Example 1 except pullulan (product of Hayashibara Shoji Inc., product name: food additive pullulan) with a molecular weight of 200,000 was used in place of the HPC to make the composition shown in Table 2.

### (Example 5)

The film-form preparation of Example 5 was obtained using the same procedure as in Example 1 except previously sized melatonin particles A were used in place of the ketoprofen particles to make the composition shown in Table 2.

### (Example 6)

The film-form preparation of Example 6 was obtained using the same procedure as in Example 1 except HPMC (product of Shin-Etsu Chemical Co., Ltd., product name: TC-5E) with a molecular weight of 16,000, a hydroxypropoxy group-substitution degree of 7.0 to 12.0%, and a methoxy group-substitution degree of 28.0 to 30.0% was used in place of the HPC, and previously sized melatonin particles A were used in place of the ketoprofen particles to make the composition shown in Table 2.

### (Example 7)

The film-form preparation of Example 7 was obtained using the same procedure as in Example 1 except previously sized indomethacin particles were used in place of the ketoprofen particles to make the composition shown in Table 2.

### (Example 8)

The film-form preparation of Example 8 was obtained using the same procedure as in Example 1 except HPMC (product of Shin-Etsu Chemical Co., Ltd., product name: TC-5E) with a molecular weight of 16,000, a hydroxypropoxy group-substitution degree of 7.0 to 12.0%, and a methoxy group-substitution degree of 28.0 to 30.0% was used in place of the HPC, and previously sized indomethacin particles were used in place of the ketoprofen particles to make the composition shown in Table 2.

**Table 2**

| Component | Examples (parts by weight) | | | | | | | |
|---|---|---|---|---|---|---|---|---|
| | 1 | 2 | 3 | 4 | 5 | 6 | 7 | 8 |
| HPC | 6.7 | - | - | - | 6.7 | - | 6.7 | - |
| PVP | - | 6.7 | - | - | - | - | - | - |
| HPMC | - | - | 6.7 | - | - | 6.7 | - | 6.7 |
| Pullulan | - | - | - | 6.7 | - | - | - | - |
| PEG400 | 0.4 | 0.4 | 0.4 | 0.4 | 0.4 | 0.4 | 0.4 | 0.4 |
| Ketoprofen particles | 3.0 | 3.0 | 3.0 | 3.0 | - | - | - | - |
| Melatonin particles A | - | - | - | - | 3.0 | 3.0 | - | - |
| Indomethacin particles | - | - | | - | - | - | 3.0 | 3.0 |
| Distilled water | 18.6 | 23.3 | 23.3 | 33.5 | 18.6 | 23.3 | 18.6 | 23.3 |

### (Example 9)

After 0.4 parts by weight of polyethylene glycol (PEG400) was added to 18.6 parts by weight of distilled water and stirred well, 6.7 parts by weight of HPC (product of Nippon Soda Co., Ltd., brand name: Nisso HPC SSL) with a molecular weight of approximately 30,000 and a hydroxypropoxy group-substitution degree of 53.4 to 77.5% was added, stirred, and dissolved using a rolling mixer. Then 3.0 parts by weight of previously sized melatonin particles B were added and dispersed by sonication to prepare a liquid dispersion of the drug. After the liquid dispersion of the drug was adequately degassed, it was spread onto a polyester release film and dried to prepare a film with a thickness of approximately 70 µm. The resulting film was cut into 4 cm² rectangles to obtain the film-form preparation of Example 9.

### (Examples 10 to 12)

The film-form preparations of Examples 10 to 12 were obtained using the same procedure as in Example 9 except the content of HPC and melatonin particles A were as shown in Table 3.

**Table 3**

| Component | Examples (parts by weight) | | | |
|---|---|---|---|---|
| | 9 | 10 | 11 | 12 |
| HPC | 6.7 | 3.6 | 8.6 | 9.5 |
| PEG400 | 0.4 | 0.4 | 0.4 | 0.4 |
| Melatonin particles A | - | 6.0 | 1.0 | 0.1 |
| Melatonin particles B | 3.0 | - | - | - |
| Distilled water | 18.6 | 15.0 | 20.3 | 20.3 |

### (Comparative Example 1)

First 0.4 parts by weight of polyethylene glycol (PEG400), 3.0 parts by weight of previously sized ketoprofen particles, and 12.2 parts by weight of ethanol were added to 6.7 parts by weight of HPC (product of Nippon Soda Co., Ltd., brand name: Nisso HPC SSL) with a molecular weight of approximately 30,000 and a hydroxypropoxy group-substitution degree of 53.4 to 77.5%, and the mixture was stirred and dissolved using a rolling mixer. After the liquid dispersion of the drug was adequately degassed, it was spread onto a polyester release film and dried to prepare a film with a thickness of approximately 70 µm. The resulting film was cut into 4 cm² rectangles to obtain a film-form preparation.

### (Comparative Example 2)

A film-form preparation was obtained using the same procedure as in Comparative Example 1 except PVP (product of Wako Pure Chemical Industries Co., Ltd., reagent name: polyvinyl pyrrolidone K90) with a molecular weight of 1,050,000 to 1,200,000 was used in place of the HPC to make the composition shown in Table 4.

### (Comparative Example 3)

A film-form preparation was obtained using the same procedure as in Comparative Example 1 except previously sized melatonin particles A were used in place of the ketoprofen particles.

### (Comparative Example 4)

A film-form preparation was obtained using the same procedure as in Comparative Example 3 except PVP (product of Wako Pure Chemical Industries Co., Ltd., reagent name: polyvinyl pyrrolidone K90) with a molecular weight of 1,050,000 to 1,200,000 was used in place of the HPC to make the composition shown in Table 4.

### (Comparative Example 5)

A film-form preparation was obtained using the same procedure as in Comparative Example 1 except previously sized indomethacin particles were used in place of the ketoprofen particles, and a mixed medium of 18.6 parts by weight ethanol and 4.7 parts by weight acetone was used as the solvent.

### (Comparative Example 6)

A film-form preparation was obtained using the same procedure as in Comparative Example 5 except PVP (product of Wako Pure Chemical Industries Co., Ltd., reagent name: polyvinyl pyrrolidone K90) with a molecular weight of 1,050,000 to 1,200,000 was used in place of the HPC to make the composition shown in Table 4.

### (Comparative Example 7)

First 0.4 parts by weight of polyethylene glycol (PEG400) was added to 15.0 parts by weight of distilled water and stirred well. Then 3.6 parts by weight of HPC (product of Nippon Soda Co., Ltd., brand name: Nisso HPC SSL) with a molecular weight of approximately 30,000 and a hydroxypropoxy group-substitution degree of 53.4 to 77.5% was added thereto, and the mixture was stirred and dissolved using a rolling mixer. Then 6.0 parts by weight of previously sized melatonin particles C were added thereto and dispersed by sonication to prepare a liquid dispersion of the drug. After the liquid dispersion of the drug was adequately degassed, it was spread onto a polyester release film and dried to prepare a film with a thickness of approximately 70 µm. The resulting film was cut into 4 cm² rectangles to obtain the film-form preparation of Comparative Example 7.

**Table 4**

| Component | Comparative Examples (parts by weight) | | | | | | |
|---|---|---|---|---|---|---|---|
| | 1 | 2 | 3 | 4 | 5 | 6 | 7 |
| HPC | 6.7 | - | 6.7 | - | 6.7 | - | 6.7 |
| PVP | - | 6.7 | - | 6.7 | - | 6.7 | - |
| PEG400 | 0.4 | 0.4 | 0.4 | 0.4 | 0.4 | 0.4 | 0.4 |
| Ketoprofen particles | 3.0 | 3.0 | - | - | - | - | - |
| Melatonin particles A | - | - | 3.0 | 3.0 | - | - | - |
| Melatonin particles C | - | - | - | - | - | - | 3.0 |
| Indomethacin particles | - | - | - | - | 3.0 | 3.0 | - |
| Ethanol | 12.2 | 18.6 | 12.2 | 18.6 | 18.6 | 18.6 | - |
| Acetone | - | - | - | - | 4.7 | 4.7 | - |
| Distilled water | - | - | - | - | - | - | 18.6 |

### [Test Methods]

Measurements and evaluations were carried out on the film-form preparations prepared in these Examples and Comparative Examples for dissolution profile in the mouth, film strength, gummy sensation in the mouth, feel when touched by the fingers, and appearance by a peeling test, oral dissolution test, tensile strength test, tack duration test, sensory test (feel), and visual observation. The particle size of the drug particles dispersed in the film-form preparation and drug crystals deposited in the film-form preparation was measured by microscopic examination. Each test method is described below.

### (1) Oral dissolution test

First 900 mL of pH 6.8 phosphate buffer was placed in a 1000 mL low glass petri dish, a stainless steel mesh basket (Φ 4 mm) was inverted and submerged therein, and agitation was provided by a stirrer (300 rpm). The temperature of the liquid was maintained at 37 ± 2°C using a constant temperature water circulator. A test piece (4 cm²) was submerged, and concurrently a 3 cm² x 3 cm² stainless steel screen (5 mm mesh) was placed on top as a sinker. The duration from the time the test piece was submerged until the test piece had finished disintegrating was checked visually and measured with a stop watch. The measurement of each sample was repeated 3 times, and the mean was used as the oral dissolution time. The oral dissolution time was then given a score using the following criteria.
4: 0 to 10 sec
3: 10 to 15 sec
2: 15 to 20 sec
1: 20 sec or longer
Prepared film-form preparations that could not be physically released from the release film were given a score of 0.

### (2) Tensile strength test

A small, tabletop, vertical tensile test apparatus (produced by Shimadzu Corporation, EZ TEST-100M) was used following "JIS K7127 Testing Method for Tensile Properties of Plastic Films and Sheets." The film-form preparation was cut to a 12 mm x 50 mm test sample, and the test was performed after thorough drying in a desiccator. A rate of 60 mm/min was used as the draw rate. Because almost no stretching was seen in the test samples, the tensile strength at the measured yield point was used as the tensile strength value.
The test was repeated 3 times for each sample, and the mean value was recorded as the tensile strength. The tensile strength was then given a score using the following criteria.
4: 10 to 20 N
3: 5 to 10 N
2: 2 to 5 N
1: 0 to 2 N
Prepared film-form preparations that could not be physically released from the release film were given a score of 0.

### (3) Stiffness test

This test was performed following the test method of "JIS L1096 Testing Methods for Woven Fabrics, 8.19 Stiffness, 8.19.1 Method A (45° cantilever method). In this test five 20 mm x 150 mm test pieces were selected, and the short dimension of the test piece was aligned with the baseline of the scale on a smooth-surfaced, flat platform with one end having a 45° downward slope. Next, the test piece was gently slid in the direction of the slope by a suitable method, and when the center point of an edge of the test piece came into contact with the slope A, the position of the trailing edge was read on the scale. Stiffness is expressed as the length (mm) that the test piece was moved. Stiffness was determined by measuring the five test pieces both top up and bottom up, and both forward and backward, and then calculating the mean value.
For the evaluation reference values, evaluation films minus the drug particles were prepared for each film-form preparation in the Examples and Comparative Examples (Reference Examples 1 to 6 below). The stiffness of the film in each Reference Example was considered a reference value, and the following scale was established.
4: Reference value ± 10 mm
3: Reference value ± 20 mm
2: Reference value ± 30 mm
1: Reference value ± 40 mm or more
If the prepared film-form preparation could not be physically peeled off the release film, it was given a score of 0.

### (Reference Example 1)

First 0.5 parts by weight of polyethylene glycol (PEG400) and 15.0 parts by weight ethanol (99.5%) were added to 9.5 parts by weight of HPC (product of Nippon Soda Co., Ltd., brand name: Nisso HPC SSL) with a molecular weight of approximately 30,000 and a hydroxypropoxy group-substitution degree of 53.4 to 77.5%, and stirred and dissolved using a rolling mixer. After the solution was adequately degassed, it was spread onto a polyester release film and dried to prepare a film with a thickness of approximately 70 µm. The resulting film was cut into 4 cm² rectangles to obtain Evaluation Film (1).

### (Reference Examples 2 to 6)

Evaluation Films (2) to (6) were obtained by the same procedure as in Reference Example 1 except the compositions shown in Table 5 were used. The PVP, HPMC and pullulan in Table 5 are the same as those described above.

**Table 5**

| Component | Reference Examples (parts by weight) | | | | | |
|---|---|---|---|---|---|---|
| | 1 | 2 | 3 | 4 | 5 | 6 |
| HPC | 9.5 | - | 9.5 | - | - | - |
| PVP | - | 9.5 | - | 9.5 | - | - |
| HPMC | - | - | - | - | 9.5 | - |
| Pullulan | - | - | - | - | - | 9.5 |
| PEG400 | 0.5 | 0.5 | 0.5 | 0.5 | 0.5 | 0.5 |
| Ethanol | 15.0 | 23.3 | - | - | - | - |
| Distilled water | - | - | 18.6 | 23.3 | 23.3 | 23.3 |
| Evaluation film | (1) | (2) | (3) | (4) | (5) | (6) |

### (4) Tack duration test

The test was performed under the environment shown in Figure 2 using a rheometer (SUN SCIENTIFIC, CR-2000). First, a 12 mm diameter test piece 2c was adhered to a 12 mm diameter probe 2a with double-sided tape 2b. Separately, a piece of rubber 2e was mounted on the test platform 2f, and a collagen film 2d soaked with water was placed thereon. Then 200 µL of purified water was applied to the test piece, the probe 2a with the test piece 2c adhered thereto was lowered, placed in contact with the top of the collagen film 2d, and then raised. At that time, the tack duration after the initial tack, which was obtained when the probe 2a released from the collagen film 2d, was measured using slide caliper from recording paper. The criteria were as follows.
4: 0 to 2 mm
3: 2 to 3 mm
2: 3 to 4 mm
1: 4 mm or more
In the case of the Comparative Examples in which the prepared film-form preparation could not be peeled off from the polyethylene terephthalate release film, the film-form preparation was cut together with the release film, and the release film side was adhered to the probe with double-sided tape. Then, the tack duration was measured in the same manner.

### (5) Sensory test (feel)

The cut film-form preparations from the Examples and Comparative Examples were evaluated for the unpleasant sensation of a sticky sensation on the surface by actually tracing a circle thereon with the fingers for 5 sec. The criteria were as follows.
4: No a sticky sensation
3: Slightly sticky but not unpleasant
2: Unpleasant sticky sensation
1: Very sticky, and film remains on the fingers.

### (6) Appearance (visual)

The film-form preparations cut from the Examples and Comparative Examples were evaluated visually for film uniformity. The criteria were as follows.
4: The film is uniform
3: Fine deposits of crystals or fine aggregates of particles are visible in some places
2: Large deposits of crystals or large aggregates of particles are visible in some places
1: Deposits of crystals or aggregates of particles are visible in the majority of places

### (7) Particle size of drug particles

The particle size of the drug particles or deposited drug crystals in the film base material was measured in the film-form preparations of the Examples and Comparative Examples using a microscope (product of Keyence Corp., model VHX-600). First 200 drug particles or deposited drug crystals were measured, and then the Mass-median-diameter (D50) was calculated therefrom.

Table 6 shows the results of the oral dissolution test, tensile strength test, stiffness test, tack duration test, sensory test (feel), appearance (visual), and average particle size of the drug particles for the preparations of Examples 1 to 12 and Comparative Examples 1 to 7.
In addition, the release properties from the polyethylene terephthalate release film were evaluated at the time of film preparation, and the results are also shown in Table 6. The criteria were as follows.
4: Can be peeled off easily
3: Can be peeled off
2: Can be peeled off with some effort
1: Can be peeled off with effort, but film tears
0: Cannot be peeled off at all
The scores from these 7 items were totaled, and a relative evaluation of the film-form preparations from the Examples and Comparative Examples was performed based on the total scores.

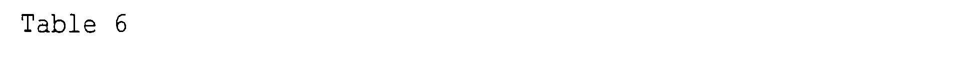

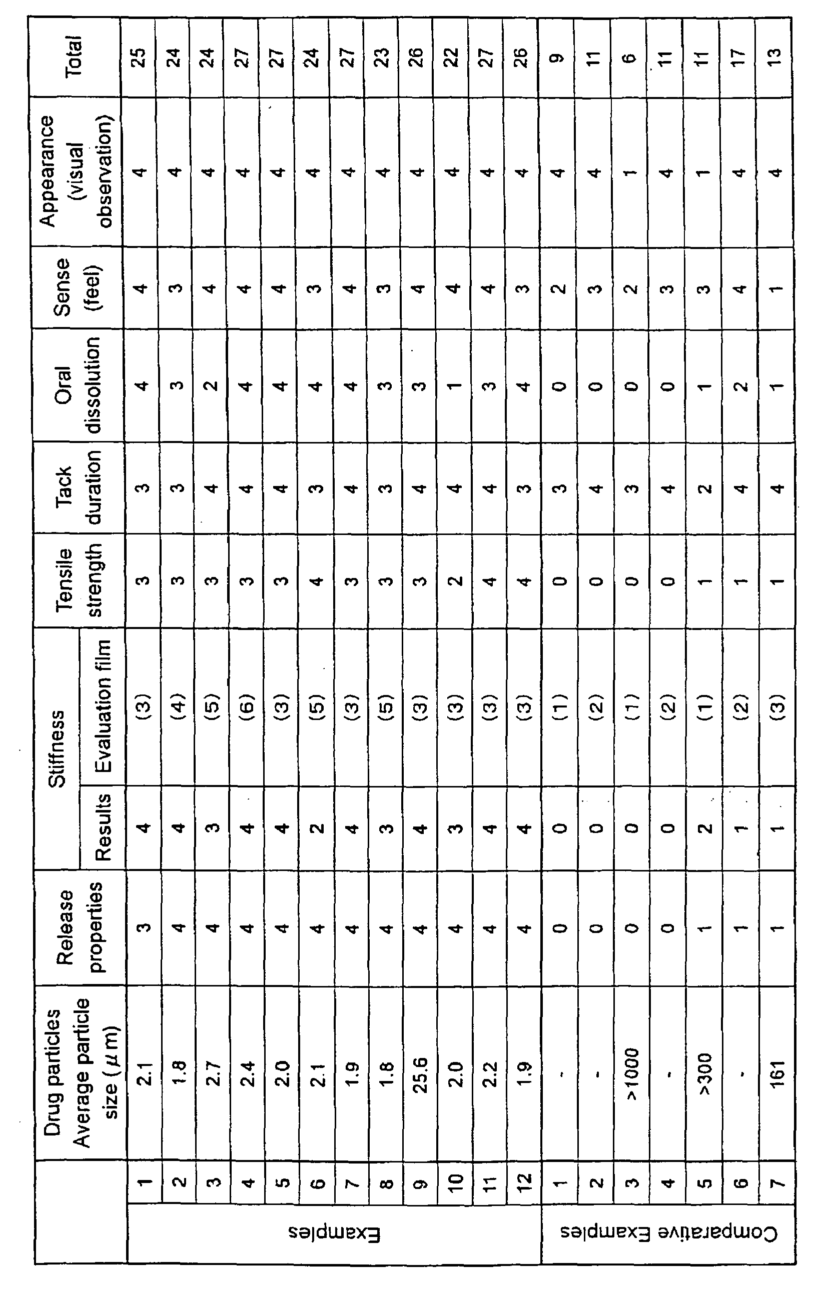

As shown in Table 6, all drug particles in the film-form preparations of the Examples are contained in a particulate state, and the average particle sizes ranged from 1.8 to 25.6 µm. Furthermore, the evaluation results for all were satisfactory, and the total scores ranged from 22 to 27 points. Although the score for the oral dissolution test in Example 10 was a "1", the content of the water-insoluble drug particles (melatonin particles A) in relation to water was greater than in the other Examples, and the drop in dissolution properties can be attributed to that.
On the other hand, the drug particles in the film-form preparations of Comparative Examples 1 to 6 were contained in a dissolved or recrystallized state. Although the drug particles were in a particulate state in the film-form preparation of Comparative Example 7, the average particle size was 161 µm. The total scores ranged from 6 to 17.

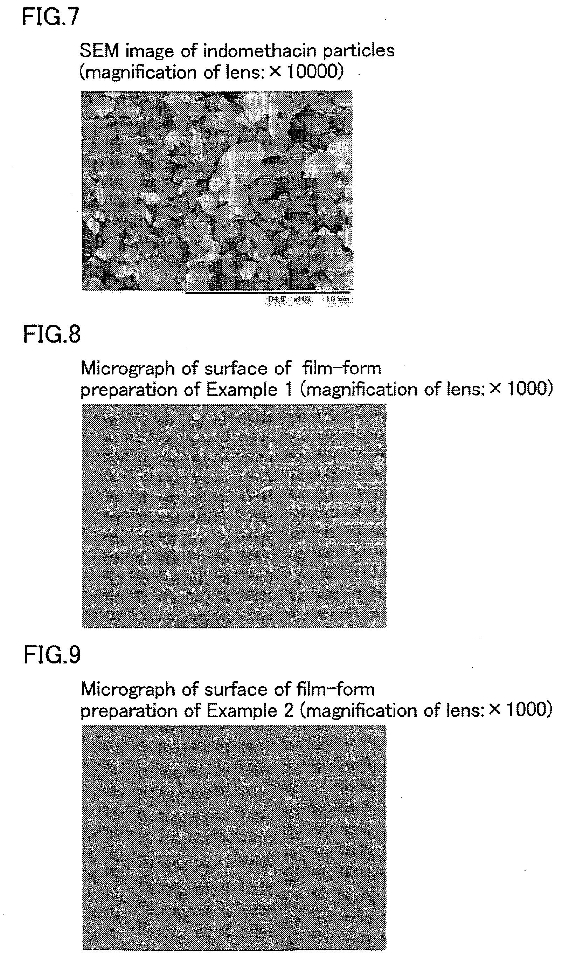

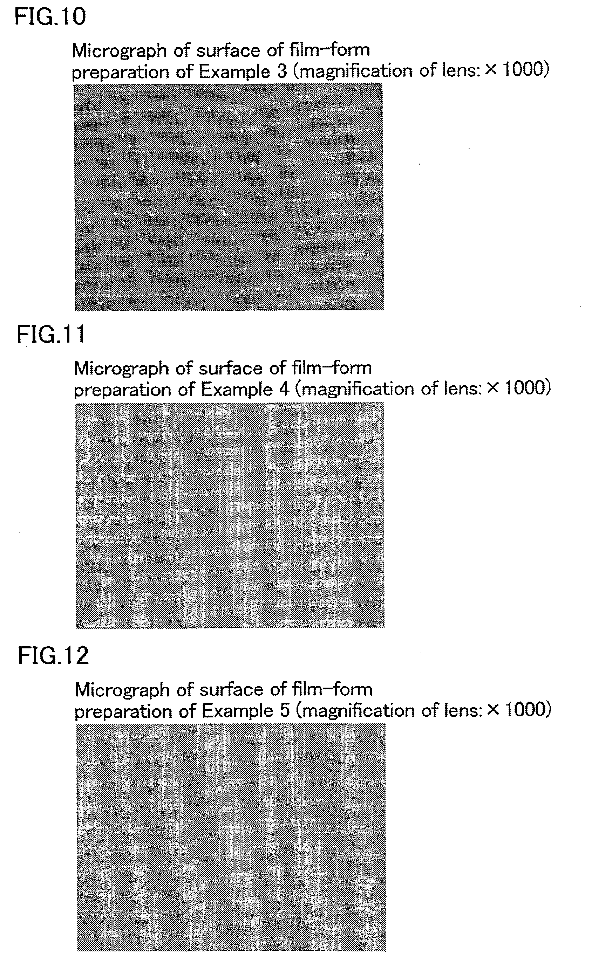

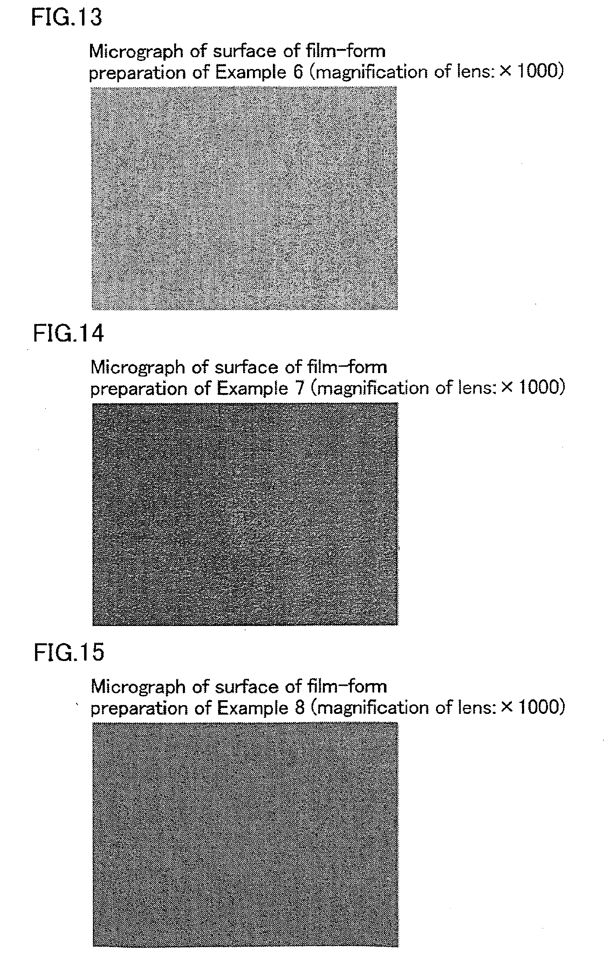

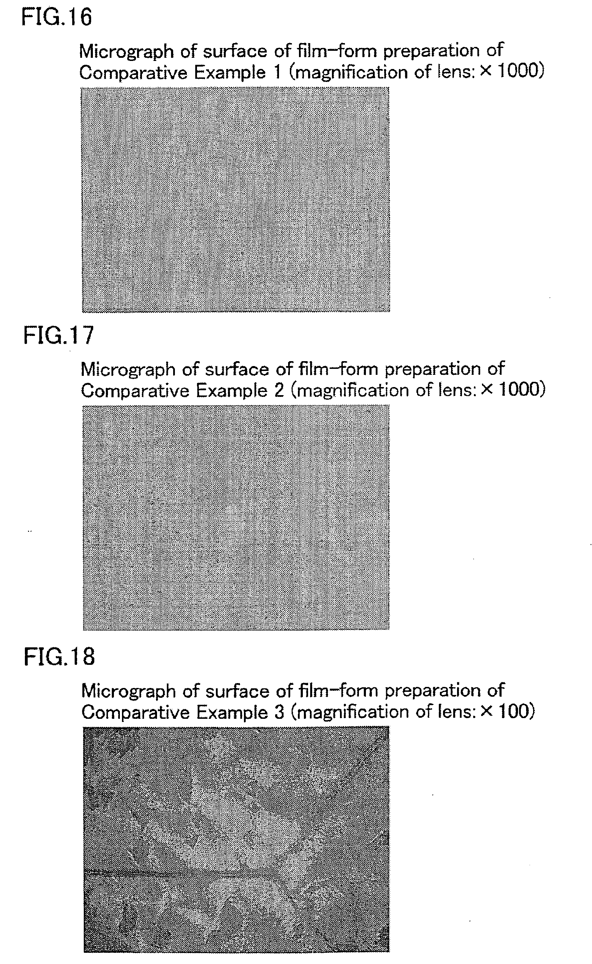

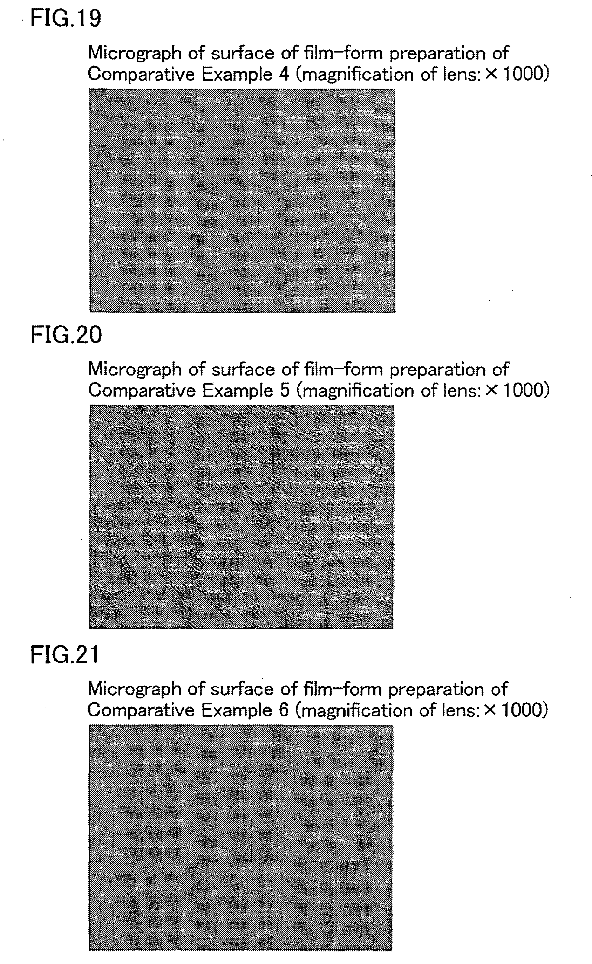

FIGS. 8 to 15 show surface micrographs of the film-form preparations of Examples 1 to 8, and FIGS. 16 to 21 show surface micrographs of the film-form preparations of Comparative Examples 1 to 6.

### INDUSTRIAL APPLICABILITY

The film-form preparation of the present invention can stably contain a sufficient quantity of drug expressing a rapid dissolution profile in the mouth because the drug particles are dispersed in a particulate state, and can have sufficient film strength, a satisfactory sensation when touched by the fingers, film appearance, and the like.
Therefore, with the film-form preparation of the present invention the drug particles do not need to be coated, e.g., encapsulated in microcapsules, to achieve the desired sustained-release capability of the drug.
Furthermore, the production method for the film-form preparation of the present invention enables the drug particles to be dispersed and carried in the film-form preparation without the need to dissolve the same in solution, and therefore a film-form preparation containing the drug in a particulate state can be produced efficiently, and the size and shape of the drug particles can be controlled thereby.

### EXPLANATION OF SYMBOLS

- 1a: Drug particles
- 1b: Base material
- 2a: Probe
- 2b: Double-sided tape
- 2c: Test piece
- 2d: Collagen film
- 2e: Rubber
- 2f: Test platform

## Claims

1. A film-form preparation comprising:
a water-soluble edible polymer; and
water-insoluble drug particles,
wherein an average particle size of the drug particles is 0.1 to 60 µm.

2. The film-form preparation according to claim 1, wherein a particle size of the drug particles is 0.1 to 30 µm.

3. The film-form preparation according to claim 1 or 2, wherein the edible polymer is a solid at normal temperatures.

4. The film-form preparation according to claim 1, 2, or 3, wherein the edible polymer is at least one type selected from the group consisting of polyvinyl pyrrolidone, hydroxypropyl cellulose, hydroxypropyl methylcellulose, and pullulan.

5. The film-form preparation according to claim 4, wherein a molecular weight of the polyvinyl pyrrolidone ranges from 2,500 to 3,000,000.

6. The film-form preparation according to claim 4, wherein a molecular weight of the hydroxypropyl cellulose ranges from 10,000 to 1,200,000.

7. The film-form preparation according to claim 4, wherein the hydroxypropyl cellulose has a hydroxypropoxy group-substitution degree of 50 to 100%.

8. The film-form preparation according to claim 4, wherein a molecular weight of the hydroxypropyl methylcellulose ranges from 10,000 to 1,500,000.

9. The film-form preparation according to claim 4, wherein the hydroxypropyl methylcellulose has a hydroxypropoxy group-substitution degree of 4 to 32%, and a methoxy group-substitution degree of 16 to 30%.

10. The film-form preparation according to claim 4, wherein a molecular weight of the pullulan ranges from 10,000 to 2,000,000.

11. A method for producing a film-form preparation including a water-soluble edible polymer and water-insoluble drug particles,
the drug particles having an average particle size of 0.1 to 60 µm, and the method comprising:
preparing a liquid dispersion of a drug containing the edible polymer, the drug particles, and water;
forming the liquid dispersion of the drug into a thin layer; and
drying the thin layer.
